# EUROPEAN PATENT APPLICATION

(11) **EP 4 763 831 A1**
(43) Date of publication of application: **24.06.2026**
(21) Application number: 24869537.1
(22) Date of filing: 09.01.2024
(51) Int. Cl.: C07C 211/63, C08F 226/02, C08F 220/52, C02F 103/10, C02F 1/56

(54) **PERFLUOROALKYL-SUBSTITUTED ALLYL QUATERNARY AMMONIUM SALT AND PREPARATION METHOD THEREFOR, COPOLYMER AS WELL AS PREPARATION METHOD THEREFOR AND USE THEREOF, AND WASTEWATER TREATMENT METHOD**

(30) Priority: 26.09.2023 CN 202311252219
(71) Applicant: PetroChina Company Limited, Dongcheng District Beijing 100007 (CN)
(72) Inventor: TANG, Quanwu, Beijing 100007 (CN); LI, Jing, Beijing 100007 (CN); FU, Jingqiang, Beijing 100007 (CN); WEN, Ming, Beijing 100007 (CN); LIN, Dong, Beijing 100007 (CN); YI, Chang, Beijing 100007 (CN); LIU, Yuan, Beijing 100007 (CN); CHEN, Tianxin, Beijing 100007 (CN); WANG, Yue, Beijing 100007 (CN); ZHAO, Liang, Beijing 100007 (CN)
(74) Representative: Ström & Gulliksson AB
(86) International application number: PCT/CN2024/071296
(87) International publication number: WO 2025/065965

(57) **Abstract**

The present invention relates to the technical field of industrial wastewater treatment in natural gas purification, and disclosed are a perfluoroalkyl-substituted allyl quaternary ammonium salt and a preparation method therefor, a copolymer as well as a preparation method therefor and a use thereof, and a wastewater treatment method. The quaternary ammonium salt has a structure represented by 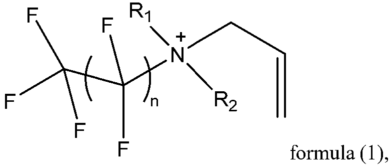 wherein R1 and R2 are each independently selected from methyl or ethyl; and n is an integer from 4 to 11. The quaternary ammonium salt is obtained by a nucleophilic substitution reaction between allylamine or a derivative thereof and perfluoroalkyl halide. The copolymer of the present invention contains a structural unit A from acrylamide and a structural unit B from the quaternary ammonium salt, and is obtained by a polymerization reaction between the acrylamide and the quaternary ammonium salt monomer. The copolymer of the present invention has relatively good salt resistance and relatively strong hydrophobic association, and can be well adapted to high-salinity industrial wastewater treatment or impurity removal in the field of natural gas purification.

## Description

### CROSS-REFERENCE TO RELATED APPLICATIONS

This application claims the benefit of Chinese Patent Application No. 202311252219.6 filed on September 26, 2023, the content of which is specifically and entirely incorporated herein by reference.

### TECHNICAL FIELD

The present disclosure relates to the technical field of industrial wastewater treatment technology in natural gas purification, and more specifically, to a perfluoroalkyl-substituted allyl quaternary ammonium salt and its preparation method, a copolymer and its preparation method and a use, and a method for treating wastewater.

### BACKGROUND

Due to China's active promotion of the green transformation of the energy structure, natural gas plays a crucial role as a green, efficient, and low-carbon fossil energy source. It is well known that high-salt organic industrial wastewater is generated during the exploration, development, production, collection, transportation, and purification processing of sulfur-containing natural gas, shale gas, and tight gas. In particular, in recent years, the intensive development of deep and ultra-deep sulfur-containing natural gas has led to an increasing total discharge volume of high-salt organic wastewater from the natural gas industry. For example, the tail gas oxidation absorption process has been gradually applied to natural gas purification. This process involves directly incinerating various sulfides in the tail gas of the sulfur recovery unit of a natural gas purification plant, converting them into SO₂, and then using highly selective organic amines to absorb SO₂ after cooling. The organic amine absorption solution can be regenerated and recycled at high temperatures, while the high-concentration SO₂ gas is returned to the sulfur recovery unit. However, the amine liquid purification stage of this process discharges a large amount of high-sulfate organic amine industrial wastewater, which urgently needs treatment.

Coagulation-flocculation methods are widely used in the treatment of industrial wastewater from natural gas purification. The key to this method lies in using suitable flocculants. Organic high-molecular-weight, inorganic, microbial, and composite flocculants are commonly used flocculants, among which polyacrylamide polymer flocculants are the most widely studied and applied. However, for industrial wastewater from natural gas purification with complex components, high sulfate content, and high organic amine content, conventional cationic polyacrylamide flocculants often fail to achieve good flocculation treatment effects due to poor salt resistance-they tend to undergo molecular chain curling under high salt conditions, resulting in loss of bridging ability.

### SUMMARY

The object of the present disclosure is to overcome the problems of conventional cationic polyacrylamide flocculants with poor salt resistance, tendency to curl molecular chains under high salt conditions, loss of bridging ability, and inability to achieve good flocculation treatment effects, by providing a perfluoroalkyl-substituted allyl quaternary ammonium salt, its preparation method, copolymer, preparation method, a use, and a method for treating wastewater.

To achieve the above object, a first aspect of the present disclosure provides a perfluoroalkyl-substituted allyl quaternary ammonium salt, wherein the quaternary ammonium salt has a structure as shown in Formula (1), 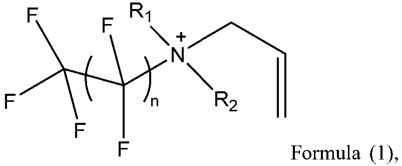 wherein R₁ and R₂ are each independently selected from methyl or ethyl; n is an integer from 4 to 11.

A second aspect of the present disclosure provides a method for preparing a perfluoroalkyl-substituted allyl quaternary ammonium salt, including: performing a nucleophilic substitution reaction between N,N-dimethylallylamine or N,N-diethylallylamine and perfluoroalkyl halide to obtain the perfluoroalkyl-substituted allyl quaternary ammonium salt, wherein the molar ratio of N,N-dimethylallylamine or N,N-diethylallylamine to perfluoroalkyl halide is 1:1.2-1.5.

A third aspect of the present disclosure provides a perfluoroalkyl-substituted allyl quaternary ammonium salt prepared by the method provided in the second aspect of the present disclosure.

A fourth aspect of the present disclosure provides a copolymer, including: a structural unit A from acrylamide and a structural unit B from perfluoroalkyl-substituted allyl quaternary ammonium salt, wherein the perfluoroalkyl-substituted allyl quaternary ammonium salt is the perfluoroalkyl-substituted allyl quaternary ammonium salt described in the present disclosure, and the molar ratio of the structural unit A: structural unit B is 196-199:1-4.

A fifth aspect of the present disclosure provides a method for preparing a copolymer, including: performing a polymerization reaction between acrylamide and a fluorine-containing cationic hydrophobic monomer in the presence of an initiator and a protective gas to obtain the copolymer.

A sixth aspect of the present disclosure provides a copolymer prepared by the method provided in the fifth aspect of the present disclosure.

A seventh aspect of the present disclosure provides a use of the copolymer as a flocculant in wastewater treatment.

Preferably, the wastewater is industrial wastewater from natural gas purification.

An eighth aspect of the present disclosure provides a method for treating industrial wastewater from natural gas purification, wherein a flocculant is added to the industrial wastewater from natural gas purification and stirred;
wherein, the flocculant is the copolymer described above.

Preferably, the industrial wastewater from natural gas purification is an organic amine wastewater containing 1-4% sulfate.

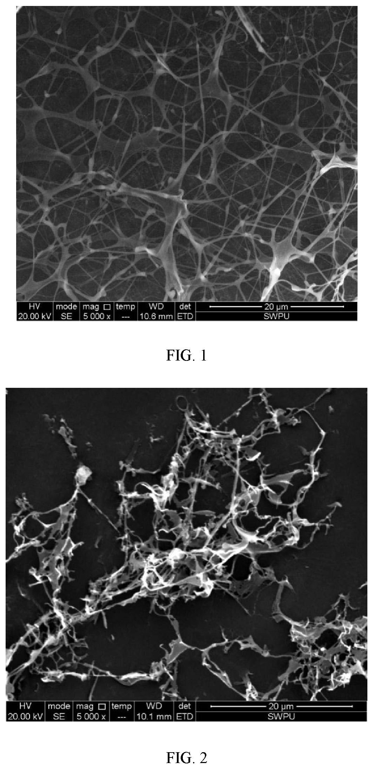

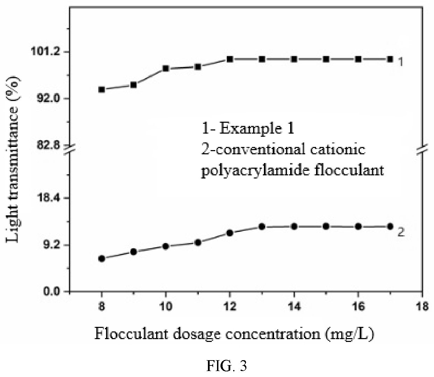

By the above technical solution, the copolymer of the present disclosure introduces a fluorine-containing cationic hydrophobic portion into the polyacrylamide. Since the fluorine-containing hydrophobic groups can aggregate, the copolymer molecules of the present disclosure to undergo intramolecular and intermolecular association in an aqueous solution under the action of electrostatic forces, hydrogen bonding, or van der Waals forces according to different concentrations. And when the critical association concentration is reached, the copolymer molecules associate to form an aggregate structure, forming a huge three-dimensional network structure, and the apparent viscosity of the solution is greatly increased. By introducing fluorinated hydrophobic groups, the flocculation time of the flocculant can be shortened and the flocculation effect can be improved. The copolymer of the present disclosure and the conventional cationic polyacrylamide flocculant were respectively used as flocculants and added to 4% sodium sulfate solutions, and the structural changes of the two flocculants in the salt solution are compared, and the results are shown in Figs. 1 and 2. It can be seen from Fig. 1 that the copolymer of the present disclosure still maintains a three-dimensional network structure in 4% sodium sulfate solution, as characterized by cryo-scanning electron microscopy, showing a strong hydrophobic association effect, and has a good bridging ability. It can be seen from Fig. 2 that conventional cationic polyacrylamide flocculants undergo molecular chain curling under high salt conditions, forming a flaky solution structure, and their bridging ability is seriously weakened. The copolymer flocculant of the present disclosure and the conventional cationic polyacrylamide flocculant were respectively used in the treatment of diatomaceous earth suspension simulating high-salt wastewater (containing 4% sodium sulfate), and the light transmittance of the supernatant of the simulated high-salt wastewater is determined by a spectrophotometer, and the flocculation treatment effect of the two flocculants on the simulated high-salt wastewater is compared within a certain dosage concentration range. The results are shown in Fig. 3. It can be seen from Fig. 3 that the light transmittance of the simulated high-salt wastewater treated with the copolymer flocculant of the present disclosure can reach more than 93%, and when the concentration of the copolymer flocculant is 12mg/L, the light transmittance of the supernatant reaches a maximum of 99.75%. This is because the copolymer flocculant not only has a positive charge that can neutralize the charge of diatomaceous earth suspension, leading to coalescence, but also exhibits a strong hydrophobic association effect with outstanding bridging ability under sulfate conditions, and promotes the formation of large flocs to better flocculate and settle the diatomaceous earth suspension in the simulated high-salt wastewater. However, the light transmittance of the supernatant of the simulated high-salt wastewater treated with the conventional cationic polyacrylamide flocculant is relatively poor, and with the increase of the dosage of the conventional cationic polyacrylamide flocculant, the increase in the light transmittance of the supernatant is not significant, indicating that the conventional cationic polyacrylamide flocculant has a poor flocculation effect on the flocculation removal of diatomaceous earth suspension in simulated high-salt wastewater, because the molecular chain of the conventional cationic polyacrylamide flocculant undergoes coiling under high salt, and the positive charge is embedded in the curled molecular chain. At the same time, the bridging ability is also seriously weakened, and it cannot effectively flocculate and settle the diatomaceous earth suspension in the simulated high-salt wastewater.

Furthermore, the perfluoroalkyl-substituted allyl quaternary ammonium salt synthesized in the present disclosure, as a fluorine-containing cationic hydrophobic monomer, exhibits surface activity and can be well dispersed in water without the need for additional small-molecule surfactants to solubilize the hydrophobic monomer, thereby avoiding the use of small-molecule surfactants. The post-treatment of the resulting copolymer is easy and does not pollute the environment. The preparation of the copolymer in the present disclosure employs an aqueous binary copolymerization method, which features a simple preparation process, easy operation, convenient control, and the ability to directly adjust the feed ratio of the quaternary ammonium salt to change the cationic degree of the copolymer without introducing other cationic monomers.

### BRIEF DESCRIPTION OF THE DRAWINGS

Figure 1 shows a cryo-scanning electron micrograph of the copolymer prepared in Example 1-1 of the present disclosure in 4% sodium sulfate solution.
Figure 2 shows a cryo-scanning electron micrograph of a commercially available cationic polyacrylamide flocculant in 4% sodium sulfate solution.
Figure 3 shows the light transmittance of the supernatant after flocculation and sedimentation of the diatomaceous earth suspension simulating high-salt wastewater (containing 4% sodium sulfate) at different dosages of the copolymer prepared in Example 1-1 of the present disclosure and a commercially available conventional cationic polyacrylamide flocculant.

### DETAILED DESCRIPTION OF THE EMBODIMENTS

The terminals and any value of the ranges disclosed herein are not limited to the precise ranges or values; such ranges or values shall be comprehended as including the values adjacent to the ranges or values. As for numerical ranges, the endpoint values of the various ranges, the endpoint values and the individual point values of the various ranges, and the individual point values may be combined with one another to produce one or more new numerical ranges, which should be deemed to have been specifically disclosed herein.

The following provides a detailed description of the specific embodiments of the disclosure in conjunction with the accompanying drawings. It should be understood that the specific embodiments described herein are only used for illustrating and explaining the disclosure and are not intended to limit the disclosure.

A first aspect of the present disclosure provides a perfluoroalkyl-substituted allyl quaternary ammonium salt, wherein the quaternary ammonium salt has a structure as shown in Formula (1), 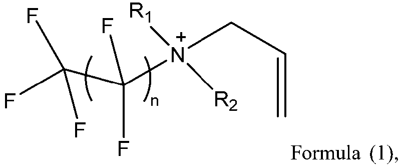 wherein R₁ and R₂ are each independently selected from methyl or ethyl; n is an integer from 4 to 11.

According to the present disclosure, the quaternary ammonium salt has surface activity and can be well dispersed in water without the need for additional small-molecule surfactants to solubilize the quaternary ammonium salt, thereby avoiding the use of small-molecule surfactants. The quaternary ammonium salt of the present invention is subjected to a polymerization reaction with acrylamide as a fluorine-containing cationic hydrophobic monomer to obtain a copolymer as a flocculant in industrial wastewater treatment. On one hand, since the quaternary ammonium salt of the present disclosure has surface activity, no additional small-molecule surfactant is required for solubilization during the polymerization reaction, making the post-treatment of the obtained copolymer easy and not polluting the environment. On the other hand, the quaternary ammonium salt of the present disclosure contains a fluorine-containing cation, and when polymerized with acrylamide, the cationic degree of the copolymer can be changed directly by adjusting the feed ratio of the quaternary ammonium salt without introducing other cationic monomers.

According to the present disclosure, only in the presence of an initiator, to enable the quaternary ammonium salt as a fluorine-containing cationic hydrophobic monomer to polymerize smoothly with acrylamide and to obtain a copolymer with good salt resistance, and to have good flocculation effect when treating industrial wastewater, preferably, a critical micelle concentration of the quaternary ammonium salt is 0.3-7 mmol/L.

According to the present disclosure, preferably, in formula (1), n is 6, 7, or 9.

In the present disclosure, the method for preparing the quaternary ammonium salt can adopt the preparation method provided in second aspect of the present disclosure.

In the present disclosure, the composition and structure of the hydrophobic associative cationic flocculant can be determined by characterization means such as infrared spectroscopy, or can be determined by the feeding amount of each material during the preparation process.

A second aspect of the present disclosure provides a method for preparing a perfluoroalkyl-substituted allyl quaternary ammonium salt, including: performing a nucleophilic substitution reaction between N,N-dimethylallylamine or N,N-diethylallylamine and perfluoroalkyl halide to obtain the perfluoroalkyl-substituted allyl quaternary ammonium salt, wherein the molar ratio of N,N-dimethylallylamine or N,N-diethylallylamine to perfluoroalkyl halide is 1:1.2-1.5.

In some embodiments of the present disclosure, the molar ratio of N,N-dimethylallylamine to perfluoroalkyl iodide is 1:1.3.

According to the present disclosure, the solvent for the nucleophilic substitution reaction is acetone and/or 1,4-dioxane.

In some embodiments of the present disclosure, the solvent for the nucleophilic substitution reaction is acetone.

According to the present disclosure, to ensure the completion of the nucleophilic substitution reaction and obtain the corresponding quaternary ammonium salt, preferably, a temperature of the nucleophilic substitution reaction is 35-55°C and a time is 6-24 h.

Furthermore, preferably, the temperature of the nucleophilic substitution reaction is 40-50°C and the time is 8-12 h.

In some embodiments of the present disclosure, the temperature of the nucleophilic substitution reaction is 40°C and the time is 12 h.

According to the present disclosure, to enable the obtained copolymer to undergo electrostatic neutralization, adsorption bridging, and other interactions with most negatively charged colloidal particles in wastewater, and to adapt well to the treatment of high-salt industrial wastewater or impurity removal in the natural gas purification field, while avoiding molecular chain coiling and weakening of bridging ability of flocculant copolymers under high salt conditions, preferably, the perfluoroalkyl halide is selected from one or more of perfluoroheptyl iodide, perfluorooctyl iodide, perfluorodecyl iodide.

In some embodiments of the present disclosure, the perfluoroalkyl iodide is perfluoroheptyl iodide, perfluorodecyl iodide, or perfluorooctyl iodide.

According to the present disclosure, after the completion of the nucleophilic substitution reaction, the method further includes the following post-treatment steps: repeatedly washing the solid powder product obtained from the nucleophilic substitution reaction with a solvent and drying at a constant temperature until constant weight.

In some embodiments of the present disclosure, after the nucleophilic substitution reaction, the washing solvent is diethyl ether and the drying temperature is 35°C.

A third aspect of the present disclosure provides a perfluoroalkyl-substituted allyl quaternary ammonium salt obtained by the preparation method of the present disclosure.

A fourth aspect of the present disclosure provides a copolymer, including: a structural unit A from acrylamide and a structural unit B from perfluoroalkyl-substituted allyl quaternary ammonium salt, wherein the perfluoroalkyl-substituted allyl quaternary ammonium salt is the perfluoroalkyl-substituted allyl quaternary ammonium salt described in the present disclosure, and the molar ratio of the structural unit A: structural unit B is 196-199:1-4.

Preferably, the molar ratio of structural unit A to structural unit B is 197-199:1-3.

According to the present disclosure, to enable the copolymer to undergo electrostatic neutralization, adsorption bridging, and other interactions with most negatively charged colloidal particles in wastewater, and to adapt well to the treatment of high-salt industrial wastewater or impurity removal in the natural gas purification field, while avoiding molecular chain curling and weakening of bridging ability of flocculant copolymers under high salt conditions, preferably, the structural unit A in the copolymer has the structure as shown in formula (2), and structural unit B has the structure as shown in formula (3), Formula (2), and the structural unit B has a structure as shown in Formula (3),

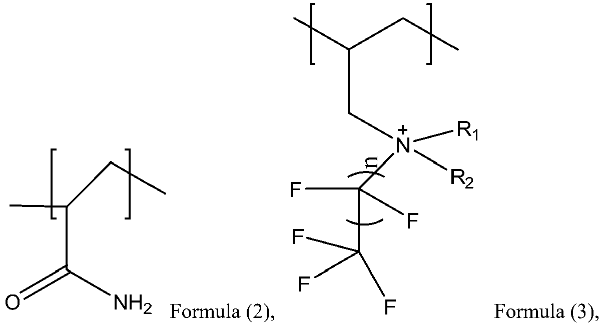

wherein R₁ and R₂ are each independently selected from methyl or ethyl; n is an integer from 4 to 11, preferably 6, 7, or 9.

In some embodiments of the present disclosure, the molar ratio of structural unit A to structural unit B is 197:3, 989:11, 1989:11, 199:1, 247:3, 1983:17, 4971:29, 989:11, 1987:11, or 497:3, and any value in the range formed by any two numerical values.

In some embodiments of the present disclosure, R₁ and R₂ are independently selected from methyl, and n is 6, 7, or 9.

According to the present disclosure, to make the association effect have a significant effect on the bridging ability of the polymer flocculant, preferably, the copolymer has a weight-average molecular weight of 2×10⁶ to 6.5×10⁶ g/mol and a critical association concentration of 0.1-0.3%.

In some embodiments of the present disclosure, the weight-average molecular weight of the copolymer is 6.5×10⁶ g/mol, 4×10⁶ g/mol, 3.5×10⁶ g/mol, 3×10⁶ g/mol, 5×10⁶ g/mol, 2×10⁶ g/mol, 5.5×10⁶ g/mol, or 1×10⁶ g/mol, and any value in the range formed by any two numerical values; and the critical association concentration is 0.1%, 0.21%, 0.25%, 0.28%, 0.13%, 0.18%, or 0.3%, and any value in the range formed by any two numerical values.

The copolymer of the present disclosure introduces a fluorine-containing cationic hydrophobic portion into the polyacrylamide. Since the fluorine-containing hydrophobic groups can aggregate, the copolymer molecules of the present disclosure to undergo intramolecular and intermolecular association in an aqueous solution under the action of electrostatic forces, hydrogen bonding, or van der Waals forces according to different concentrations. And when the critical association concentration is reached, the copolymer molecules associate to form an aggregate structure, forming a huge three-dimensional network structure, and the apparent viscosity of the solution is greatly increased. By introducing fluorinated hydrophobic groups, the flocculation time of the flocculant can be shortened and the flocculation effect can be improved. The polymer of the present disclosure is used as a flocculant and added to a 4% sodium sulfate solution. The structural changes of the flocculant in the salt solution are observed. The results are shown in Figure 1. It can be seen from Fig. 1 that the copolymer flocculant still has a three-dimensional network structure in a 4% sodium sulfate solution, showing a strong hydrophobic association effect and good bridging ability.

In the present disclosure, the method for preparing the copolymer can employ the method provided in the fifth aspect of the present disclosure.

In the present disclosure, the composition and structure of the copolymer can be determined by characterization means such as infrared spectroscopy, or by the feeding amount of each material during the preparation process.

A fifth aspect of the present disclosure provides a method for preparing a copolymer, including: performing a polymerization reaction between acrylamide and a fluorine-containing cationic hydrophobic monomer in the presence of an initiator and a protective gas to obtain the copolymer.

According to the present disclosure, the polymerization reaction is carried out in an aqueous solution, with deionized water as the solvent.

Preferably, the solute is acrylamide and fluorine-containing cationic hydrophobic monomer, and the mass fraction of the solute in the polymerization reaction solution is controlled to be 20-25%.

In some embodiments of the present disclosure, the solute is acrylamide and fluorine-containing cationic hydrophobic monomer, and the mass fractions of the solute in the polymerization reaction solution are 25%, 23%, 22% or 20%, and any value in the range formed by any two numerical values.

According to the preparation method of the present disclosure, to ensure the smooth progress of the polymerization reaction and obtain the copolymer, preferably, the initiator is selected from one or more of sodium persulfate, sodium sulfite, and 2,2'-azobis(2-methylpropionamidine) dihydrochloride; wherein, based on the total weight of acrylamide and fluorine-containing cationic hydrophobic monomer, the mass percentage of the initiator is 0.05-0.4 wt%.

Preferably, the mass percentage of the initiator is 0.1-0.3 wt%.

In some embodiments of the present disclosure, the initiator is a composite initiator in a mass ratio of sodium persulfate, sodium sulfite, and 2,2'-azobis(2-methylpropionamidine) dihydrochloride of 2:1:7.

In some embodiments of the present disclosure, the mass percentages of the initiator are 0.3%, 0.2% or 0.1%, and any value in the range formed by any two numerical values.

According to the preparation method of the present disclosure, to enable the obtained copolymer used as a flocculant, to undergo electrostatic neutralization, adsorption bridging, and other effects with most negatively charged colloidal particles in wastewater, and to adapt well to the treatment of high-salt industrial wastewater or impurity removal in the natural gas purification field, and to avoid the molecular chain curling and weakening of bridging ability of the flocculant polymer molecule under high salt conditions, preferably, the fluorine-containing cationic hydrophobic monomer is the above perfluoroalkyl-substituted allyl quaternary ammonium salt.

According to the preparation method of the present disclosure, to improve the water solubility of the copolymer, preferably, the molar ratio of acrylamide to the fluorine-containing cationic hydrophobic monomer is 100:0.5-2.5.

Further preferably, the molar ratio of acrylamide to the fluorine-containing cationic hydrophobic monomer is 100:0.7-2.

In some embodiments of the present disclosure, the molar ratio of acrylamide to the fluorine-containing cationic hydrophobic monomer is 100:2, 100:1.5, 100:1 or 100:0.7, and any value in the range formed by any two numerical values.

According to the preparation method of the present disclosure, to ensure the smooth progress of the polymerization reaction and obtain the copolymer, preferably, a temperature of the polymerization reaction is 45-55°C and a time is 4-8 h.

Preferably, the temperature of the polymerization reaction is 45-50°C and the time is 5-7 h.

In some embodiments of the present disclosure, the temperature of the polymerization reaction is 50°C or 45°C, and the reaction time is 5 or 7 h.

According to the preparation method of the present disclosure, to maintain the polymerization initiator system at an appropriate pH to facilitate the polymerization reaction, preferably, the pH of the solution of the polymerization reaction is adjusted to 6-9 using sodium hydroxide. Preferably, the pH of the polymerization reaction solution is adjusted to 7-8 using sodium hydroxide.

In some embodiments of the present disclosure, the pH of the polymerization reaction solution is 7 or 8.

According to the preparation method of the present disclosure, after the completion of the polymerization reaction, the method further includes post-treatment steps: washing the reaction product with a solvent several times, placing the washed product in a vacuum oven to dry to a constant weight, and grinding it into a powder.

Preferably, the washing solvent is ethanol, and the vacuum drying temperature is 55°C.

In some embodiments of the present disclosure, the fluorine-containing cationic hydrophobic monomers are perfluoroheptyl dimethylallyl ammonium iodide, perfluorooctyl dimethylallyl ammonium iodide, or perfluorodecyl dimethylallyl ammonium iodide.

By the preparation method provided in the fifth aspect of the present disclosure, the aforementioned copolymer can be obtained. According to the above statements, when the polymer of the present disclosure is used as a flocculant in industrial wastewater treatment, due to the positive charge carried by the copolymer molecules themselves, they can neutralize the negatively charged colloidal particles in industrial wastewater, leading to coalescence. Moreover, the fluorinated hydrophobic groups within the copolymer molecules tend to aggregate in aqueous solutions, exhibiting strong hydrophobic association effects. Under sulfate conditions, the bridging ability is prominent, promoting the formation of large flocs, thereby better flocculating and settling suspensions in high-salt wastewater. In addition, the fluorine-containing cationic hydrophobic monomer synthesized by the present disclosure has surface activity, can be well dispersed into water, does not need to add other small-molecule surfactants to solubilize the hydrophobic monomer, avoids the use of small-molecule surfactants, and the obtained copolymer is easy to post-treat and will not cause pollution to the environment. The preparation of the copolymer of the present disclosure adopts the method of aqueous binary copolymerization method. The preparation process is simple, easy to operate, easy to control, and the cationic degree of the copolymer can be changed directly by adjusting the proportion of fluorine-containing cationic hydrophobic monomers in the monomer composition, and there is no need to introduce other cationic monomers.

In a sixth aspect, the present disclosure provides a copolymer prepared by the preparation method provided in the fifth aspect of the present disclosure.

The seventh aspect of the present disclosure provides a use of the above-mentioned polymer as a flocculant in wastewater treatment.

Preferably, the wastewater is a industrial wastewater from natural gas purification.

The eighth aspect of the present disclosure provides a method for treating industrial wastewater from natural gas purification, wherein a flocculant is added to the industrial wastewater from natural gas purification and stirred; wherein, the flocculant is the copolymer described above.

Preferably, the industrial wastewater from natural gas purification is an organic amine wastewater containing 1-4% sulfate.

Using the copolymer of the present disclosure as a flocculant, flocculation treatment is performed on the diatomaceous earth suspension simulating high-salt wastewater (containing 4% sodium sulfate). And the light transmittance of the supernatant of the treated simulated high-salt wastewater is determined by a spectrophotometer. The flocculation results are shown in Fig. 3. It can be seen from Fig. 3 that under the same experimental conditions, compared with the conventional cationic polyacrylamide flocculant, the light transmittance of the simulated high-salt wastewater treated with the copolymer of the present disclosure can reach more than 93%. Moreover, when the copolymer dosing concentration was 12mg/L, the light transmittance of the supernatant reached a maximum of 99.75%. However, the light transmittance of the supernatant treated with conventional cationic polyacrylamide flocculant was relatively poor, and the light transmittance of the supernatant did not increase significantly with the increase of the dosage of conventional cationic polyacrylamide flocculant.

The following Examples are used to illustrate the technical scheme of the present disclosure, wherein the raw material and reagents used are commercially available and the room temperature refers to 15-35°C.

N,N-dimethylallylamine, 98% purity, purchased from Shanghai Aladdin Biochemical Technology Co., Ltd.

N,N-diethylallylamine, purity greater than 98%, purchased from Shanghai Aladdin Biochemical Technology Co., Ltd.

Perfluoroheptyl iodide, purity greater than 98%, purchased from Shanghai Aladdin Biochemical Technology Co., Ltd.

Perfluorooctyl iodide, 98% purity, purchased from Shanghai Aladdin Biochemical Technology Co., Ltd.

Perfluorodecyl iodide, 98% purity, purchased from Shanghai Aladdin Biochemical Technology Co., Ltd.

Sodium persulfate, analytical grade, purchased from Shanghai Macklin Biochemical Technology Co., Ltd.

Sodium sulfite, analytical grade, purchased from Shanghai Macklin Biochemical Technology Co., Ltd.

2,2'-azobis(2-methylpropionamidine) dihydrochloride, purity 97%, purchased from Shanghai Macklin Biochemical Technology Co., Ltd.

Conventional cationic polyacrylamide flocculant, industrial grade, purchased from China National Pharmaceutical Group Chemical Reagents Co., Ltd.

The structure and composition of the perfluoroalkyl-substituted allyl quaternary ammonium salt were determined by infrared spectroscopy.

The structure and composition of the copolymer molecules were determined by the feeding amount of each material during the preparation process.

The weight-average molecular weight of the copolymer molecules was determined by gel permeation chromatography.

The apparent viscosity (or other physicochemical property parameters) of the copolymer molecules was determined by the Brookfield DV-II rotary viscometer.

### Example 1

### (1) Preparation of fluorine-containing cationic hydrophobic monomer perfluoroheptyl dimethylallyl ammonium iodide

Dissolve 1 mol of N,N-dimethylallylamine in 3 mol of acetone and transfer it to a three-neck flask; heat under stirring to 40°C, and then add 1.3 mol of perfluoroheptyl iodide dropwise to the three-neck flask. After the addition was complete, stir for reaction for 12 h, suction filter to obtain a solid powder product. Wash repeatedly with diethyl ether three times and suction filter to obtain the washed solid powder product. Finally, dry the washed solid powder product in a constant temperature oven at 35°C until constant weight to obtain the target product I perfluoroheptyl dimethylallyl ammonium iodide.

The surface tension of target product I was measured, and the critical micelle concentration of target product I was 7 mmol/L.

### (2) Preparation of copolymer I

Add the deionized water to the polymerization reactor, add the perfluoroheptyl dimethylallyl ammonium iodide prepared in step (1) and acrylamide successively under stirring, and control the mass fraction of the solute and the pH of the solution. Subsequently, after introducing nitrogen for 30 minutes, heat the solution. Then add a composite initiator consisting of sodium persulfate, sodium sulfite, and 2,2'-azobis(2-methylpropionamidine) dihydrochloride in a mass ratio of 2:1:7 to carry out the polymerization reaction. Wash the obtained product with ethanol several times, place it in a vacuum oven at 55°C to dry until constant weight, and grind it into a powder to obtain the copolymer I.

The weight-average molecular weight of copolymer I was determined by gel permeation chromatography, and the results are shown in Table 1.

By adjusting the reaction conditions in Example 1(2), copolymers I-1, I-2, I-3 and I-4 can be obtained. The reaction conditions and the composition and physicochemical property parameters of the corresponding copolymers obtained are shown in Table 1.

**Table 1**

| Number | Example I-1 | Example I-2 | Example I-3 | Example I-4 |
|---|---|---|---|---|
| Molar ratio of acrylamide to perfluoroheptyl dimethylallyl ammonium iodide | 100:2 | 100:1.5 | 100:1 | 100:0.7 |
| Mass fraction of solute in the polymerization solution (%) | 25 | 23 | 22 | 20 |
| Mass percentage of the initiator (based on total weight of acrylamide and fluorine-containing cationic hydrophobic monomer) (%) | 0.3 | 0.2 | 0.2 | 0.1 |
| Polymerization temperature (°C) | 50 | 50 | 45 | 45 |
| Polymerization reaction time (h) | 7 | 7 | 5 | 5 |
| pH of the polymerization reaction solution | 8 | 8 | 7 | 7 |
| Molar ratio of structural unit A to structural unit B | 197:3 | 989:11 | 1987:11 | 199:1 |
| Weight-average molecular weight (g/mol) | 6.5×10⁶ | 4×10⁶ | 3.5×10⁶ | 3×10⁶ |
| Critical association concentration (%) | 0.1 | 0.21 | 0.25 | 0.28 |
| Apparent viscosity at critical association concentration (mPa·s) | 112 | 94 | 91 | 85 |

### Example 2

### Preparation of fluorine-containing cationic hydrophobic monomer perfluorooctyl dimethylallyl ammonium iodide

Dissolve 1 mol of N,N-dimethylallylamine in 3 mol of acetone and transfer to a three-neck flask; heat under stirring to 40°C, and then add 1.3 mol of perfluorooctyl iodide dropwise to the three-neck flask. After the addition was complete, stir for reaction for 12 h, suction filter to obtain a solid powder product. Wash repeatedly with diethyl ether three times and suction filter to obtain the washed solid powder product. Finally, dry the washed solid powder product in a constant temperature oven at 35°C until constant weight to obtain the target product II perfluorooctyl dimethylallyl ammonium iodide.

The surface tension of target product II was measured, and the critical micelle concentration of target product II was 2.5 mmol/L.

### (2) Preparation of copolymer II

Add deionized water to the polymerization reactor, add the perfluorooctyl dimethylallyl ammonium iodide prepared in step (1) and acrylamide successively under stirring, and control the mass fraction of the solute and the pH of the solution. Subsequently, after introducing nitrogen for 30 minutes, heat the solution. Then add a composite initiator consisting of sodium persulfate, sodium sulfite, and 2,2'-azobis(2-methylpropionamidine) dihydrochloride in a mass ratio of 2:1:7 to carry out the polymerization reaction. Wash the obtained product with ethanol several times, place it in a vacuum oven at 55°C to dry until constant weight, and grind it into a powder to obtain the copolymer II.

The weight-average molecular weight of copolymer II was determined by gel permeation chromatography, and the results are shown in Table 2.

By adjusting the reaction conditions in Example 2 (2), copolymer II-1, II-2, II-3, and II-4 can be obtained. The reaction conditions and the composition and physicochemical property parameters of the corresponding copolymers obtained are shown in Table 2.

**Table 2**

| Number | Example II-1 | Example II-2 | Example II-3 | Example II-4 |
|---|---|---|---|---|
| Molar ratio of acrylamide to perfluorooctyl dimethylallyl ammonium iodide | 100:2 | 100:1.5 | 100:1 | 100:0.7 |
| Mass fraction of solute in the polymerization solution (%) | 25 | 23 | 22 | 20 |
| Mass percentage of the initiator (based on total weight of acrylamide and fluorine-containing cationic hydrophobic monomer) (%) | 0.3 | 0.2 | 0.2 | 0.1 |
| Polymerization temperature (°C) | 50 | 50 | 45 | 45 |
| Polymerization reaction time (h) | 7 | 7 | 5 | 5 |
| pH of the polymerization reaction solution | 8 | 8 | 7 | 7 |
| Molar ratio of structural unit A to structural unit B | 197:3 | 247:3 | 1983:17 | 4971:29 |
| Weight-average molecular weight (g/mol) | 5×10⁶ | 4×10⁶ | 3.5×10⁶ | 2×10⁶ |
| Critical association concentration (%) | 0.13 | 0.21 | 0.25 | 0.28 |
| Apparent viscosity at critical association concentration (mPa·s) | 125 | 108 | 97 | 88 |

### Example 3

### (1) Preparation of fluorine-containing cationic hydrophobic monomer perfluorodecyl dimethylallyl ammonium iodide

Dissolve 1 mol of N,N-dimethylallylamine in 3 mol of acetone and transfer to a three-neck flask; heat under stirring to 40°C, and then add 1.3 mol of perfluorodecyl iodide dropwise to the three-neck flask. After the addition was complete, stir for reaction for 12 h, and suction filter to obtain a solid powder product. Wash repeatedly with diethyl ether three times and suction filter to obtain the washed solid powder product. Finally, dry the washed solid powder product in a constant temperature oven at 35°C until constant weight to obtain the target product III perfluorodecyl dimethylallyl ammonium iodide.

The surface tension of the target product III was measured, and the critical micelle concentration of the target product III was 0.3 mmol/L.

### (2) Preparation of copolymer III

Add deionized water to the polymerization reactor, add the perfluorodecyl dimethylallyl ammonium iodide prepared in step (1) and acrylamide successively under stirring, and control the mass fraction of the solute and the pH of the solution. Subsequently, after introducing nitrogen for 30 minutes, heat the solution. Then, add a composite initiator consisting of sodium persulfate, sodium sulfite, and 2,2'-azobis(2-methylpropionamidine) dihydrochloride in a mass ratio of 2:1:7 to carry out the polymerization reaction. Wash the obtained product with ethanol several times, place it in a vacuum oven at 55°C to dry until constant weight, and grind it into a powder to obtain the copolymer III.

The weight-average molecular weight of copolymer III was determined by gel permeation chromatography, and the results are shown in Table 3.

By adjusting the reaction conditions in Example 3(2), copolymer III-1, III-2, III-3, and III-4 can be obtained. The reaction conditions and the composition and physicochemical property parameters of the corresponding copolymers obtained are shown in Table 3.

**Table 3**

| Number | Example III-1 | Example III-2 | Example III-3 | Example III-4 |
|---|---|---|---|---|
| Molar ratio of acrylamide to perfluorodecyl dimethylallyl ammonium iodide | 100:2 | 100:1.5 | 100:1 | 100:0.7 |
| Mass percentage of solute in polymerization solution (%) | 25 | 23 | 22 | 20 |
| Mass percentage of the initiator (based on total weight of acrylamide and fluorine-containing cationic hydrophobic monomer) (%) | 0.3 | 0.2 | 0.2 | 0.1 |
| Polymerization temperature (°C) | 50 | 50 | 45 | 45 |
| Polymerization reaction time (h) | 7 | 7 | 5 | 5 |
| pH of the polymerization reaction solution | 8 | 8 | 7 | 7 |
| Molar ratio of structural unit A to structural unit B | 197:3 | 247:3 | 1983:17 | 4971:29 |
| Weight-average molecular weight (g/mol) | 5.5×10⁶ | 4×10⁶ | 3×10⁶ | 1×10⁶ |
| Critical association concentration (%) | 0.1 | 0.18 | 0.25 | 0.3 |
| Apparent viscosity at critical association concentration (mPa·s) | 136 | 121 | 115 | 91 |

### Test Example 1

The copolymer I-1 prepared by Example 1-1 of the present disclosure and the conventional cationic polyacrylamide flocculant are added to 4% sodium sulfate solution, and the structure of the copolymer molecules in the solution is determined by cryo-scanning electron microscopy, and the results are shown in Fig. 1 and Fig. 2. It can be seen from Fig. 1 that the copolymer of the present disclosure has a three-dimensional network structure in a 4% sodium sulfate solution, showing a strong hydrophobic association effect and good bridging ability, while it can be seen from Fig. 2 that the conventional cationic polyacrylamide flocculant undergoes molecular chain curling under high salt, forming a flaky solution structure, and its bridging ability is seriously weakened.

### Test Example 2

At 25°C, the copolymer I-1 prepared by Example 1-1 was used as the flocculant, and the conventional cationic polyacrylamide flocculant was added to the diatomaceous earth suspension simulating high-salt wastewater (containing 4% sodium sulfate) respectively, and the treatment solution containing different mass concentrations of flocculant was obtained. After sufficient stirring and standing for a period of time, the supernatant was taken, and the light transmittance of the supernatant was measured by a spectrophotometer to evaluate the flocculation effect of each flocculant, and the results are shown in Fig. 3.

It can be seen from Fig. 3 that the light transmittance of the simulated high-salt wastewater treated with the copolymer flocculant of the present disclosure can reach more than 93%, and when the dosage concentration of the copolymer flocculant is 12 mg/L, the light transmittance of the supernatant reaches a maximum of 99.75%. This is because the copolymer flocculant not only has a positive charge that can neutralize the charge of diatomaceous earth suspension, leading to coalescence, but also exhibits a strong hydrophobic association effect with outstanding bridging ability under sulfate conditions, and promotes the formation of large flocs to better flocculate and settle the diatomaceous earth suspension in the simulated high-salt wastewater. However, the light transmittance of the supernatant of the simulated high-salt wastewater treated with the conventional cationic polyacrylamide flocculant is relatively poor, and with the increase of the dosage of the conventional cationic polyacrylamide flocculant, the increase in the light transmittance of the supernatant is not significant, indicating that the conventional cationic polyacrylamide flocculant has a poor flocculation effect on the flocculation removal of diatomaceous earth suspension in simulated high-salt wastewater, because the molecular chain of the conventional cationic polyacrylamide flocculant undergoes coiling under high salt, and the positive charge is embedded in the curled molecular chain. At the same time, the bridging ability is also seriously weakened, and it cannot effectively flocculate and settle the diatomaceous earth suspension in the simulated high-salt wastewater.

Through the above examples and test examples, it can be seen that the copolymer of the present disclosure still has a three-dimensional network structure under high salt. And when applied to the treatment of high-salt industrial wastewater or the removal of impurities in the field of natural gas purification, the copolymer of the present disclosure not only has a positive charge that can neutralize the charge of diatomaceous earth suspension, leading to coalescence, but also exhibits a strong hydrophobic association effect with outstanding bridging ability. Furthermore, the fluorine-containing cationic hydrophobic monomer synthesized in the present disclosure exhibits surface activity and can be well dispersed in water without the need for additional small-molecule surfactants to solubilize the hydrophobic monomer, thereby avoiding the use of small-molecule surfactants. The post-treatment of the resulting copolymer is easy and does not pollute the environment. The preparation of the copolymer in the present disclosure employs an aqueous binary copolymerization method, which features a simple preparation process, easy operation, convenient control, and the ability to directly adjust the feed ratio of the quaternary ammonium salt to change the cationic degree of the copolymer without introducing other cationic monomers.

The above content describes the preferred embodiments of the present disclosure in detail, but the present disclosure is not limited thereto. A variety of simple modifications can be made to the technical solutions of the present disclosure within the scope of the technical concept of the present disclosure, including a combination of individual technical features in any other suitable manner; such simple modifications and combinations thereof shall also be regarded as the content disclosed by the present disclosure, each of them falls into the protection scope of the present disclosure.

## Claims

1. A perfluoroalkyl-substituted allyl quaternary ammonium salt, wherein the quaternary ammonium salt has a structure as shown in formula (1), 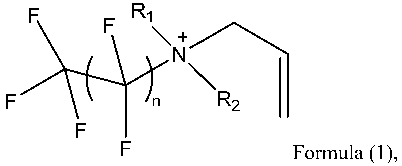 wherein, R₁ and R₂ are each independently selected from methyl or ethyl; and n is an integer from 4 to 11.

2. The quaternary ammonium salt according to claim 1, wherein a critical micelle concentration of the quaternary ammonium salt is 0.3-7 mmol/L;
preferably, n is 6, 7, or 9.

3. A method for preparing a perfluoroalkyl-substituted allyl quaternary ammonium salt, comprising: performing a nucleophilic substitution reaction between N,N-dimethylallylamine or N,N-diethylallylamine and perfluoroalkyl halide to obtain the perfluoroalkyl-substituted allyl quaternary ammonium salt, wherein the molar ratio of N,N-dimethylallylamine or N,N-diethylallylamine to perfluoroalkyl halide is 1:1.2-1.5.

4. The preparation method according to claim 3, wherein a temperature of the nucleophilic substitution reaction is 35-55°C, and a time is 6-24 h;
preferably, the temperature of the nucleophilic substitution reaction is 40-50 °C and the time is 8-12 h;
and/or, the perfluoroalkyl halide is selected from one or more of perfluoroheptyl iodide, perfluorooctyl iodide, perfluorodecyl iodide.

5. A perfluoroalkyl-substituted allyl quaternary ammonium salt prepared by the method according to claim 3 or 4.

6. A copolymer, comprising: a structural unit A from acrylamide and a structural unit B from perfluoroalkyl-substituted allyl quaternary ammonium salt, wherein the perfluoroalkyl-substituted allyl quaternary ammonium salt is the perfluoroalkyl-substituted allyl quaternary ammonium salt according to any of the claims 1-2 and 5, and the molar ratio of the structural unit A: structural unit B is 196-199:1-4.

7. The copolymer according to claim 6, wherein the structural unit A has a structure as shown in Formula (2), and the structural unit B has a structure as shown in Formula (3), 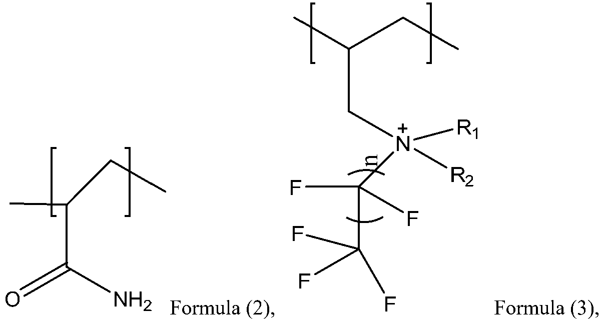
wherein R₁ and R₂ are each independently selected from methyl or ethyl; n is an integer from 4 to 11, preferably 6, 7, or 9;
preferably, the molar ratio of structural unit A to structural unit B is 197-199:1-3.

8. The copolymer according to claims 6 or 7, wherein the copolymer has a weight-average molecular weight of 2×10⁶ to 6.5×10⁶ g/mol and a critical association concentration of 0.1-0.3%.

9. A method for preparing a copolymer, comprising: performing a polymerization reaction between acrylamide and a fluorine-containing cationic hydrophobic monomer in the presence of an initiator and a protective gas to obtain the copolymer; wherein the fluorine-containing cationic hydrophobic monomer is the perfluoroalkyl-substituted allyl quaternary ammonium salt according to any one of claims 1-2 and 5.

10. The preparation method according to claim 9, wherein the initiator is selected from one or more of sodium persulfate, sodium sulfite, and 2,2'-azobis(2-methylpropionamidine) dihydrochloride;
and/or, based on the total weight of acrylamide and the fluorine-containing cationic hydrophobic monomer, the mass percentage of the initiator is 0.05-0.4 wt%, preferably 0.1-0.3 wt%.

11. The preparation method according to claim 9 or 10, wherein the molar ratio of acrylamide to the fluorine-containing cationic hydrophobic monomer is 100:0.5-2.5, preferably 100:0.7-2.

12. The preparation method according to claims 9-11, wherein a temperature of the polymerization reaction is 40-55°C, and a time is 4-8 h, preferably the temperature of the polymerization reaction is 45-50°C, and the time is 5-7 h;
and/or, the pH of the polymerization reaction is 6-9, preferably 7-8.

13. A copolymer prepared by the method according to any one of claims 9-12.

14. Use of the copolymer according to any one of claims 6-8 and 13 as a flocculant in wastewater treatment.

15. The use according to claim 14, wherein the wastewater is industrial wastewater from natural gas purification.

16. A method for treating industrial wastewater from natural gas purification, comprising mixing a flocculant with industrial wastewater from natural gas purification;
wherein the flocculant is the copolymer according to any one of claims 6-8 and 13.

17. The method according to claim 16, wherein the industrial wastewater from natural gas purification is an organic amine wastewater containing 1-4% sulfate.
